# EUROPEAN PATENT SPECIFICATION

(11) **EP 4 606 403 B1**
(45) Date of publication and mention of the grant of the patent: **02.09.2026**
(21) Application number: 25181965.2
(22) Date of filing: 04.03.2021
(51) Int. Cl.: A61M 5/30, A61M 37/00, A61M 5/315, A61M 5/20, A61M 5/31

(54) **NEEDLELESS SYRINGE**
NADELLOSE SPRITZE
SERINGUE SANS AIGUILLE

(30) Priority: 06.03.2020 KR 20200028431
(43) Date of publication of application: 27.08.2025
(62) Document number(s) of the earlier application(s) in accordance with Art. 76 EPC: 21764051.5 / 3 970 769
(73) Proprietor: Baz Biomedic Co., Ltd., Seoul 06133 (KR)
(72) Inventor: KIM, Jung Kook, 06133 Gangnam-gu, Seoul (KR); LEE, Sung Hun, 06133 Gangnam-gu, Seoul (KR); HAM, Hwi Chan, 06133 Gangnam-gu, Seoul (KR)
(74) Representative: Vossius & Partner Patentanwälte Rechtsanwälte mbB

(56) References cited:
- CN-A- 102 247 637
- US-A1- 2018 056 004

## Description

### TECHNICAL FIELD

The present invention relates to a needleless syringe, and more particularly, to a needleless syringe capable of repeatedly injecting a drug at high speed without an injection needle.

### BACKGROUND ART

In general, a syringe is a device for injecting a medicinal solution into the tissue of an organism. The syringe includes a needle inserted into the body, a syringe cylinder in which the medicinal solution is accommodated, and a piston that reciprocates inside the syringe cylinder and pushes the medicinal solution with the needle. The needle is punctured to allow a drug to be injected when injected.

Recently, in order to relieve the fear of the needle of the syringe and to prevent infection due to the needle, research and development on the syringe without the needle has been actively carried out.

However, because the existing needleless syringe is configured to inject a predetermined amount of a drug into only one part of the skin at a time, damage to the skin tissue may occur.

In addition, because discomfort such as reloading after one injection follows, there is a limitation in that the needleless syringe cannot be used to evenly inject the drug multiple times into a large area of the skin in the field of skin care and the like.

US 2018/056004 A1 discloses a device for repetitive needleless injection of a liquid into a surface. This device includes a handheld unit which includes at least a cell being fillable with the liquid. A propulsion mechanism is configured to apply a sequence of pressure pulses to the liquid. Each pulse ejects a micro jet of the liquid from the cell via an orifice between the cell and the exterior of the handheld unit with a velocity that is sufficient to enable the micro-jet to penetrate into the surface.

### DETAILED DESCRIPTION OF THE INVENTION

### TECHNICAL PROBLEM

The objective of the present invention is to provide a needleless syringe in which a predetermined amount of a drug is repeatedly injected at high speed and is evenly injected into a larger area of the skin.

### TECHNICAL SOLUTION

The present invention is defined by the appended independent claim, and preferred aspects of the present invention are defined by the appended dependent claims. In the following description parts, there is further provided a needleless injector including: a body formed in a hollow shape; a solenoid coil wound around an outer circumferential surface of the body; a cylinder coupled to the body to be in communication with an open front surface of the body and including a drug accommodating portion for accommodating a drug, and a nozzle portion for discharging the drug accommodated in the drug accommodating portion to a front; a moving magnetic body, which is long inserted into the body in a longitudinal direction and moves forward by a magnetic force generated when a current is applied to the solenoid coil; a piston, which is inserted in front of the moving magnetic body inside the body and is provided to penetrate the body and the cylinder, and moves forward by an impact force applied by the moving magnetic body when the moving magnetic body moves forward, to pressurize the drug in the drug accommodating portion toward the nozzle portion; an elastic member for a moving magnetic body, which is provided between the moving magnetic body and the piston to apply an elastic force to the moving magnetic body in a direction in which the moving magnetic body moves backward; a nozzle portion opening/closing valve, which is provided to open and close a passage hole between the nozzle portion and the drug accommodating portion, is pushed by a fluidic pressure applied by the drug from the drug accommodating portion when the piston moves forward, to open the passage hole, and when the fluidic pressure is released, the nozzle portion opening/closing valve being elastically restored to close the passage hole; and a forward/backward driving unit repeating forward and backward movement of the piston by repeating the supply and cut off of a current to the solenoid coil at a preset period, wherein the forward and backward driving unit includes: a current supply unit, which repeatedly supplies a current to the solenoid coil when the piston moves forward, to move the moving magnetic body forward, and repeatedly cuts off the current to the solenoid coil when the piston moves backward; and an elastic member of a piston, which applies an elastic force to the piston in a direction in which the piston moves backward when the supply of current of the current supply unit is cut off.

The current supply unit may cut off the current after applying the current to the solenoid coil for a first preset time, and a mass of the moving magnetic body may be 100 g or less, and the first preset time may be set to 250 ms or less.

The elastic member for the moving magnetic body may include a first coil spring, which is compressed when the moving magnetic body moves forward and applies an elastic force to the moving magnetic body in a direction in which the moving magnetic body moves backward.

The piston may have a first flange portion protruding in a radial direction from an outer circumferential surface of a front portion located inside the cylinder, and the elastic member for the piston may include a second coil spring extrapolated on the piston, both ends of the second coil spring being provided between the cylinder and the first flange portion, the second coil spring being compressed when the piston moves forward and applying an elastic force to the first flange portion in a direction in which the piston moves backward.

The piston may have a second flange portion protruding in a radial direction from an outer circumferential surface of a rear portion located inside the body, and the elastic member for the piston may include a third coil spring extrapolated on the piston, both ends of the third coil spring being provided between the body and the second flange portion, the third coil spring being compressed when the piston moves forward and applying an elastic force to the second flange portion in the direction in which the piston moves backward.

The piston may have a first flange portion protruding in a radial direction from an outer circumferential surface of a front portion located inside the cylinder, and a second flange portion protruding in a radial direction from an outer circumferential surface of a rear portion located inside the body, and the elastic member for the piston may include a second coil spring extrapolated on the piston, both ends of the second coil spring being provided between the cylinder and the first flange portion, the second coil spring being compressed when the piston moves forward and applying an elastic force to the first flange portion in a direction in which the piston moves backward, and a third coil spring extrapolated on the piston, both ends of the third coil spring being provided between the body and the second flange portion, the third coil spring being compressed when the piston moves forward and applying an elastic force to the second flange portion in the direction in which the piston moves backward.

The needleless syringe may further include a blocker provided between the piston and the cylinder and limiting a forward movement distance of the piston when the first flange portion is blocked during forward movement of the piston.

The needleless syringe may further include a ring-shaped fixed blocker fixedly installed on an inner circumferential surface of the cylinder and having a female thread formed on an inner circumferential surface of the fixed blocker, and a length adjustment blocker, which is screwed to the inner circumferential surface of the fixed blocker and in which the first flange portion is blocked when the piston moves forward and a coupling length at which the length adjustment blocker screwed to the fixed blocker is adjustable.

The drug accommodating portion may be formed in a shape of a diverging nozzle including a reduced portion whose cross-sectional area decreases toward the front and an enlarged portion extending from the reduced portion to increase the cross-sectional area again, and the reduced portion may have a drug supply hole in which the drug is supplied from an outside by a pressure difference generated during backward movement of the piston.

The nozzle portion opening/closing valve may include a ball installed in the passage hole and an elastic member installed in the nozzle portion to support the ball.

The needless syringe further includes a piston cover provided inside the cylinder, configured to cover an end of the piston and formed of a stretchable material so as to be stretched when the piston moves forward or backward.

The needleless syringe may further include a cooling chamber provided to surround an outside of the solenoid coil from an outside of the body, to absorb heat generated in the solenoid coil through a cooling fluid and to cool the solenoid coil.

In the following description parts, there is further provided a needleless syringe including: a body formed in a hollow shape; a solenoid coil wound around an outer circumferential surface of the body; a cylinder coupled to the body to be in communication with an open front surface of the body and including a drug accommodating portion for accommodating a drug, and a nozzle portion for discharging the drug accommodated in the drug accommodating portion to a front; a moving magnetic body, which is long inserted into the body in a longitudinal direction and moves forward by a magnetic force generated when a current is applied to the solenoid coil; a piston, which is inserted in front of the moving magnetic body inside the body and is provided to penetrate the body and the cylinder, and moves forward by an impact force applied by the moving magnetic body when the moving magnetic body moves forward, to pressurize the drug in the drug accommodating portion toward the nozzle portion; an elastic member for a moving magnetic body, which is provided between the moving magnetic body and the piston to apply an elastic force to the moving magnetic body in a direction in which the moving magnetic body moves backward; a nozzle portion opening/closing valve, which is provided to open and close a passage hole between the nozzle portion and the drug accommodating portion, is pushed by a fluidic pressure applied by the drug from the drug accommodating portion when the piston moves forward, to open the passage hole, and when the fluidic pressure is released, the nozzle portion opening/closing valve being elastically restored to close the passage hole; a forward/backward driving unit repeating forward and backward movement of the piston by repeating the supply and cut off of a current to the solenoid coil at a preset period; a piston cover provided inside the cylinder, configured to cover an end of the piston and formed of a stretchable material so as to be stretched when the piston moves forward or backward; and a cooling chamber provided to surround an outside of the solenoid coil from an outside of the body, to absorb heat generated in the solenoid coil through a cooling fluid and to cool the solenoid coil, wherein the forward/backward driving unit includes: a current supply unit, which repeatedly supplies a current to the solenoid coil when the piston moves forward, to move the moving magnetic body forward, and repeatedly cuts off the current to the solenoid coil when the piston moves backward; and an elastic member of a piston, which applies an elastic force to the piston in a direction in which the piston moves backward when the supply of current of the current supply unit is cut off, and the current supply unit may cut off the current after applying the current to the solenoid coil for a first preset time, and a mass of the moving magnetic body may be 100 g or less, and the first preset time may be set to 250 ms or less.

### EFFECTS OF THE INVENTION

In a needleless syringe according to the present invention, a current is supplied to a solenoid coil to generate a magnetic field so as to move a moving magnetic body and a piston forward, and when the current to the solenoid coil is cut off, an elastic member for the moving magnetic body moves the moving magnetic body backward and an elastic member for the piston moves the piston backward, so that the piston for injecting a drug by pressurizing the drug is configured to reciprocate repeatedly forward and backward and thus, a predetermined drug can be repeatedly injected at high speed.

In addition, according to the present invention, time for which the current is applied to the solenoid coil, is adjusted to minimize recoil without adding a recoil offset structure so that the structure is simple and convenience of use can be improved.

In addition, the moving magnetic body collides with the piston to move the piston forward so that the drug can be injected at higher speed by using less energy.

In addition, multiple-time injection instead of one injection can be performed when one treatment is performed on a wider range of skin such as the face in the field of skin beauty, etc.

In addition, a small amount of a drug can be automatically and repeatedly injected at high speed without the need for a user to separately load the needleless syringe.

In addition, a voltage applied to the solenoid coil and the coupling length of a length adjustment blocker are changed so that one injection amount of the drug can be adjusted.

In addition, a cooling chamber is provided around the solenoid coil so that a magnetic force can be prevented from being weakened by heat generated in the solenoid coil.

In addition, since a piston cover is provided between a cylinder and the piston so that the drug can be prevented from being deposited on an end of the piston and thus the user does not need to wipe the end of the piston.

### DESCRIPTION OF THE DRAWINGS

FIG. 1 is a longitudinal cross-sectional view showing a needleless syringe according to a first embodiment not falling into the scope of claim 1;
FIG. 2 is a view showing a forward movement state of a piston of the needleless syringe according to the first embodiment;
FIG. 3 is a view showing a backward movement state of the piston of the needleless syringe according to the first embodiment;
FIG. 4 is a view showing a nozzle portion opening/closing valve of the needleless syringe according to the first embodiment;
FIG. 5 is a graph showing an example of a current supply waveform applied to a solenoid coil of the needleless syringe according to the first embodiment;
FIG. 6 is a graph showing another example of a current supply waveform applied to the solenoid coil of the needleless syringe according to the first embodiment;
FIG. 7 is a graph showing comparison of a recoil displacement according to time when a current is applied to the solenoid coil, in the needleless syringe according to the first embodiment;
FIG. 8 is a graph showing a recoil force (Force) according to time when a current is applied to the solenoid coil, in the needleless syringe according to the first embodiment;
FIG. 9 is a longitudinal sectional view showing a configuration in which a cooling chamber is provided in a needleless syringe according to a second embodiment not falling into the scope of claim 1; and
FIG. 10 is a longitudinal cross-sectional view showing a configuration in which a piston cover is provided in a needleless syringe according to a third embodiment falling into the scope of claim 1.

### MODE OF THE INVENTION

Hereinafter, the present invention will be described with reference to the accompanying drawings.

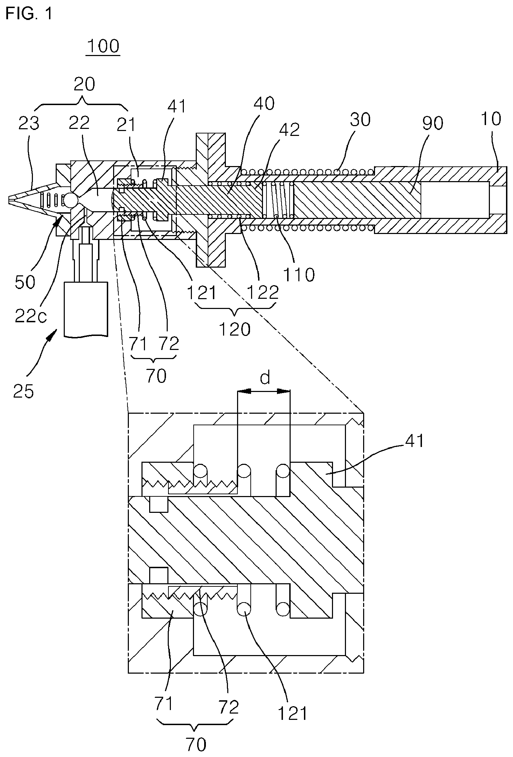

FIG. 1 is a longitudinal cross-sectional view showing a needleless syringe according to a first embodiment not falling into the scope of claim 1.

Referring to FIG. 1, a needleless syringe 100 includes a body 10, a cylinder 20, a solenoid coil 30, a moving magnetic body 90, an elastic member 110 for a moving magnetic body, a piston 40, a nozzle portion opening/closing valve 50, a forward/backward driving unit 60, and a blocker 70.

The needleless syringe 100 is an impact-type syringe in which, when the moving magnetic body 90 moves forward by a magnetic force generated by the solenoid coil 30, the moving magnetic body 90 collides with the piston 40 to move the piston 40 forward.

The body 10 is formed in a hollow shape and is formed to be elongated in a longitudinal direction. The front surface of the body 10 is formed to be opened.

The cylinder 20 is screwed to the front of the body 10. The cylinder 20 is coupled to the body 10 to be in communication with the open front surface of the body 10.

The cylinder 20 is formed in a hollow shape and is formed in such a way that a cylinder main hole 21, a drug accommodating portion 22 and a nozzle portion 23 are in communication with each other.

The cylinder main hole 21 is formed in the rear of an inner side of the cylinder 20, and a thread is formed in such a way that the front end of the body 10 is inserted into at least a part of the cylinder 20 to be screw-coupled to each other.

The drug accommodating portion 22 is formed to have a reduced cross-sectional area compared to the cylinder main hole 21. The drug accommodating portion 22 is a passage through which the piston 40 is in close contact with the drug accommodating portion 22, and is an accommodation space in which a drug is accommodated.

The drug accommodating portion 22 is formed in a shape of a diverging nozzle including a reduced portion 22a whose cross-sectional area gradually decreases toward the front, and an enlarged portion 22b that extends from the reduced portion 22a and increases in cross-sectional area again. A drug supply hole 22c for supplying the drug from the outside by a pressure difference generated when the piston 40 moves backward is formed in the reduced portion 22a. A drug filling device 25 is coupled to the drug supply hole 22c.

The nozzle portion 23 is formed to be in communication with the drug accommodating portion 22 and to have a gradually decreasing cross-sectional area, and injects the drug accommodated in the drug accommodating portion 22.

In the present embodiment, an example in which the cylinder 20 is formed by coupling a first block in which the cylinder main hole 21 and the drug accommodating portion 22 are formed and a second block in which the nozzle portion 23 is formed, will be described. However, the present invention is not limited thereto, and the first block and the second block may also be integrally formed.

The solenoid coil 30 is a coil, which is wound on the front side of an outer circumferential surface of the body 10 and to which a current is applied when the piston 40 moves forward.

The solenoid coil 30 generates a magnetic force in a direction in which the moving magnetic body 90 moves forward when a current is applied, so as to move the moving magnetic body 90 forward.

The piston 40 is long inserted into the body 10 and the cylinder 20 in the longitudinal direction and pushes the drug accommodated in the drug accommodating portion 22.

The piston 40 is provided separately from the moving magnetic body 90 inside the body 10, and is inserted in front of the moving magnetic body 90. The piston 40 moves forward by an impact force applied by the moving magnetic body 90 during forward movement of the moving magnetic body 90 to pressurize the drug in the drug accommodating portion 22 toward the nozzle portion 23.

In the piston 40, a first flange portion 41 protruding in a radial direction is formed on the outer circumferential surface of a front portion located inside the cylinder 20.

The first flange portion 41 is blocked by a length adjustment blocker 7 to be described later during the forward movement of the piston 40 so that a forward movement distance of the piston 40 is limited.

In the piston 40, a second flange portion 42 protruding in the radial direction is formed on the outer circumferential surface of a rear portion located inside the body 10.

The blocker 70 is detachably coupled between the cylinder 20 and the piston 40.

The blocker 70 includes a fixed blocker 71 coupled to and fixed to the cylinder main hole 21, and a length adjustment blocker 72 that is screwed to an inner circumferential surface of the fixed blocker 71 and adjusts a length at which the length adjustment blocker 72 is coupled to the fixed blocker 71.

The fixed blocker 71 has a female thread formed on an inner circumferential surface of the fixed blocker 71 and formed in a ring shape.

The length adjustment blocker 72 has a male thread on an outer circumferential surface of the length adjustment blocker 72 and formed in a ring shape. A predetermined interval is formed between the length adjustment blocker 72 and the piston 40, and the piston 40 may pass through the inside of the length adjustment blocker 72 to move forward and backward.

The length adjustment blocker 72 is screwed at the rear of the fixed blocker 72, and a coupling length at which the length adjustment blocker 72 is screw-coupled to the fixed blocker 72, may be adjusted differently depending on one injection amount of the drug.

As the length of the length adjustment blocker 72 screwed to the fixed blocker 71 increases, the length at which the length adjustment blocker 72 protrudes backward decreases. When the length of the length adjustment blocker 72 protruding backward decreases, a distance d between the length adjustment blocker 72 and the first flange portion 41 increases, so that a forward movement distance d of the piston 40 increases. As the forward movement distance d of the piston 40 increases, one injection amount of the drug is increased.

As the coupling length at which the length adjustment blocker 72 is screwed to the fixed blocker 71 decreases, the length at which the length adjustment blocker 72 protrudes backward increases. As the length at which the length adjustment blocker 72 protruding backward increases, the distance d between the length adjustment blocker 72 and the first flange portion 41 decreases, so that the forward movement distance d of the piston 40 decreases. As the forward movement distance d of the piston decreases, one injection amount of the drug is reduced.

Thus, the user may finely adjust one injection amount of the drug by adjusting the length at which the length adjustment blocker 72 is coupled to the fixed blocker 71.

The moving magnetic body 90 is long inserted into the body 10 in the longitudinal direction, and reciprocates forward and backward by a magnetic force generated when a current is applied to the solenoid coil 30.

The moving magnetic body 90 is not a permanent magnet, but is formed of a material which has a magnetism temporarily by a magnetic field generated when a current is applied to the solenoid coil 30 and in which the magnetism disappears when an external magnetic field disappears. An example in which the moving magnetic body 90 is an iron core, will be described.

The elastic member 110 for the moving magnetic body is long provided in the longitudinal direction of the body 10 between the moving magnetic body 90 and the piston 40 inside the body 10. The elastic member 110 for the moving magnetic body applies an elastic force to the moving magnetic body 110 in a direction in which the moving magnetic body 90 moves backward. The elastic member 110 for the moving magnetic body is a first coil spring that is compressed when the moving magnetic body 90 moves forward and applies an elastic force to the moving magnetic body 90 in the backward movement direction of the moving magnetic body 90. One end of the elastic member 110 for the moving magnetic body may be coupled to the rear end of the piston 40, and the other end of the elastic member 110 for the moving magnetic body may be coupled to the front end of the moving magnetic body 90.

The nozzle portion opening/closing valve 50 is provided to open and close a passage hole between the nozzle portion 23 and the drug accommodating portion 22. The nozzle portion opening/closing valve 50 is pushed by a fluidic pressure applied by the drug accommodated in the drug accommodating portion 22 during the forward movement of the piston 40 to open the passage hole, and is elastically restored when the fluidic pressure is released to close the passage hole.

The nozzle portion opening/closing valve 50 includes a ball 51 installed in the passage hole, and an elastic member 52 that is installed in the nozzle portion 23 to provide an elastic force in a direction of the ball 51 toward the drug accommodating portion 22. The ball 51 is formed to fit into the enlarged portion 22b. In the present embodiment, the nozzle portion opening/closing valve 50 has been described as an example of a ball valve. However, the present invention is not limited thereto, and various valves such as a duckbill valve, a plate check valve, an electric control valve, and the like may be used as the nozzle portion opening/closing valve 50.

The forward/backward driving unit may repeat the supply and cut-off of current to the solenoid coil 30 at a preset period, thereby repeating the forward movement and the backward movement of the piston 40 a plurality of times.

The forward/backward driving unit includes a current supply unit (not shown) and an elastic member 120 for the piston.

The current supply unit (not shown) applies a current to the solenoid coil 30 when the piston 40 moves forward, thereby moving the moving magnetic body 90 forward. When the piston 40 moves backward, the current supply unit (not shown) cuts off the current to the solenoid coil 30.

The current supply unit (not shown) may use any one of a capacitor (not shown) for storing a current supplied from an external power supply source and a direct current (DC) power supply unit (not shown) for supplying a current supplied from the external power supply source.

The capacitor (not shown) supplies the stored current to the solenoid coil 30 when the moving magnetic body 90 moves forward and discharges, and when the moving magnetic body 90 moves backward, the capacitor (not shown) stores the current and charges without supplying a current to the solenoid coil 30. Thus, because an electric energy applied during the forward movement of the moving magnetic body 90 is greater than the electric energy applied during the backward movement, forward movement speed may be increased.

The elastic member 120 for the piston is an elastic member that applies an elastic force to the piston 40 in a direction in which the piston 40 moves backward when the current supply unit cuts off the supply of current.

The elastic member 120 for the piston includes a second coil spring 121 and a third coil spring 122 coupled to an outer circumferential surface of the piston 40.

The second coil spring 121 is extrapolated on the piston 40, and both ends of the second coil spring 121 are provided between the cylinder 20 and the first flange portion 41. The second coil spring 121 is compressed by the first flange portion 41 when the piston 40 moves forward, and when the piston 40 moves backward, the second coil spring 121 applies an electric force to the first flange portion 41 in a direction in which the piston 40 moves backward.

The third coil spring 122 is extrapolated on the piston 40 and both ends of the third coil spring 122 are provided between the body 10 and the second flange portion 42. The second coil spring 122 is compressed by the second flange portion 42 when the piston 40 moves forward, and when the piston 40 moves backward, the third coil spring 122 applies an electric force to the second flange portion 42 in a direction in which the piston 40 moves backward.

The operation of the needleless syringe 100 according to the first embodiment configured as described above will be described as follows.

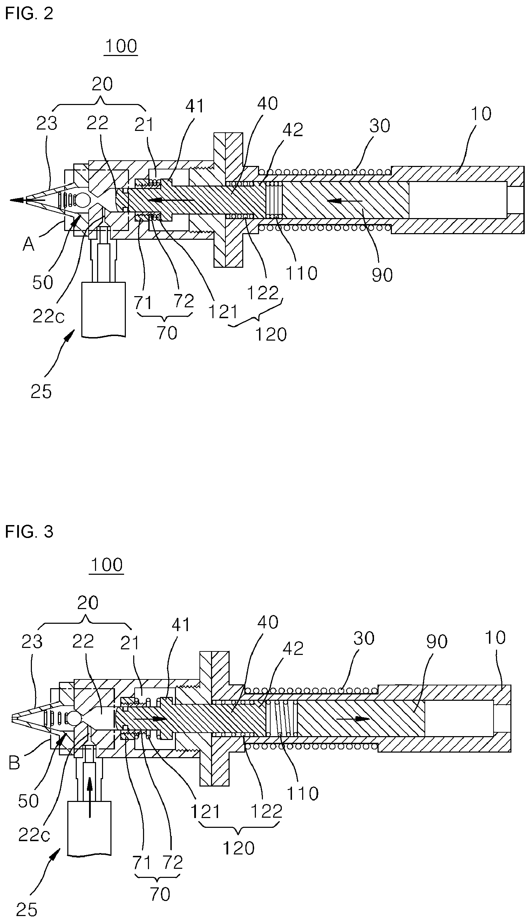

FIG. 2 is a view showing a forward movement state of the piston of the needleless syringe according to the first embodiment. FIG. 3 is a view showing a backward movement state of the piston of the needleless syringe according to the first embodiment.

Referring to FIG. 2, when the piston 40 moves forward, a current in a first preset direction is applied to the solenoid coil 30. Here, the first direction is set to a direction in which a magnetic force is generated in a direction in which the piston 40 moves forward around the solenoid coil 30.

The moving magnetic body 90 moves forward by the magnetic force generated by the solenoid coil 30.

When the moving magnetic body 90 moves forward, the elastic member 110 for the moving magnetic body is compressed.

When the moving magnetic body 90 moves forward more than a predetermined distance, the moving magnetic body 90 collides with the piston 40.

The piston 40 moves forward by an impact force applied while the moving magnetic body 90 collides with the piston 40.

The impact force is proportional to the mass and the movement velocity of the moving magnetic body 90. The movement velocity may be adjusted according to a voltage applied to the solenoid coil 30 and a movement distance of the moving magnetic body 90. If the impact force is changed by adjusting the voltage, one injection amount of the drug may be adjusted.

In the present embodiment, because the piston 40 moves forward by the impact force, the piston 40 may move forward at a higher speed compared to a case where the piston 40 and the magnetic moving body 90 move forward integrally. Thus, an electrical energy required to move the piston 40 forward can be further reduced.

When the piston 40 moves forward, the elastic member 120 for the piston is compressed. That is, when the piston 40 moves forward, the second coil spring 121 is compressed by the first flange portion 41, and the third coil spring 122 is compressed by the second flange portion 42.

The piston 40 may move forward only until the first flange portion 41 is blocked by the length adjustment blocker 72.

When the piston 40 moves forward, the piston 40 pressurizes the drug in the drug accommodating portion 22.

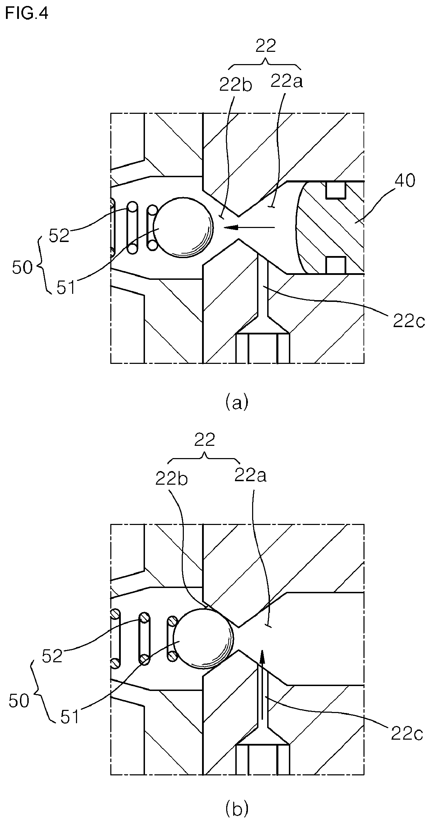

Referring to FIG. 4A, when the drug in the drug accommodating portion 22 is pressurized, the nozzle portion opening/closing valve 50 is opened by the fluidic pressure.

When the nozzle portion opening/closing valve 50 is opened, the drug in the drug accommodating portion 22 may be injected forward through the nozzle portion 23 .

When the piston 40 moves forward, as described above, because the piston 40 may only move forward until the first flange portion 41 is blocked by the length adjustment blocker 72, the forward movement distance of the piston 40 is limited.

Thus, the coupling length at which the length adjustment blocker 72 is coupled to the fixed blocker 71, is adjusted to adjust the length at which the length adjustment blocker 72 protruding backward toward the first flange portion 41 so that the forward movement distance of the piston 40 can be adjusted. By adjusting the forward movement distance of the piston 40, one injection amount of the drug may be adjusted.

That is, one injection amount of the drug is adjustable according to the magnitude of the voltage applied to the solenoid coil 30 and the forward movement distance of the piston 40.

Subsequently, the current supply unit supplies a current to the solenoid coil 30 for a first preset time Δt, and then, when the first preset time Δt elapses, the supply of current to the solenoid coil 30 is cut off.

The first preset time Δt is set to about 250 ms or less. The first preset time Δt will be described later in detail.

Referring to FIG. 3, when the piston 40 moves backward, the supply of current to the solenoid coil 30 is cut off.

When the current to the solenoid coil 30 is cut off, the magnetic field by the solenoid coil 30 disappears, and the force for moving the moving magnetic body 90 forward disappears.

The moving magnetic body 90 moves backward by an elastic restoring force of the elastic member 110 for the moving magnetic body. That is, the moving magnetic body 90 is returned to its original position by the elastic force of the elastic member 110 for the moving magnetic body.

In addition, the piston 40 moves backward to the original position by the elastic restoring force of the elastic member 120 for the piston.

Referring to FIG. 4B, when the piston 40 moves backward, the pressure in the drug accommodating portion 22 is lowered, so that the nozzle portion opening/closing valve 50 is elastically restored and closed.

In addition, when the pressure in the drug accommodating portion 22 is lowered, the drug may be filled in the drug accommodating portion 22 from the drug filling device 25 through the drug supply hole 22c. That is, when the piston 40 moves backward, the drug may be automatically filled.

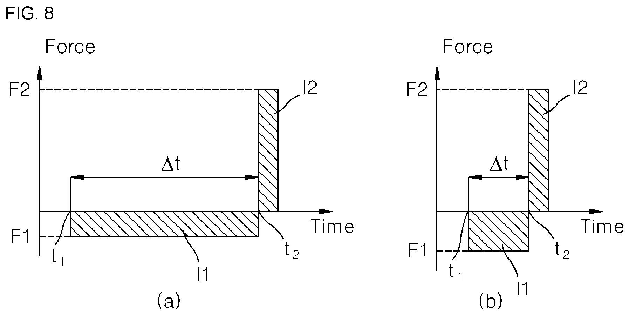

FIG. 8 is a graph showing comparison of a recoil force (Force) according to time when a current is applied to the solenoid coil, in the needleless syringe according to the first embodiment.

Meanwhile, the current supply unit supplies a current to the solenoid coil 30 for a first preset time Δt, and then cuts off the current when the first preset time Δt elapses.

The first preset time Δt is a time difference from a time t1 of applying a current to the solenoid coil 30 to a time t2 of cutting off the current to the solenoid coil 30.

The first preset time Δt is set to a time when a primary recoil impulse I1 generated when the moving magnetic body 90 moves forward and a secondary recoil impulse I2 generated when the moving magnetic body 90 collides with the piston 40 can be offset each other.

When the moving magnetic body 90 moves forward, according to the law of action and reaction, primary recoil occurs in the needleless syringe 100 or the hand of the user who holds the needleless syringe 100 in the backward movement direction opposite to the forward movement direction.

When the moving magnetic body 90 collides with the piston 40, according to the law of action and reaction, secondary recoil occurs in the needleless syringe 100 or the hand of the user who holds the needleless syringe 100 in the forward movement direction.

Referring to FIG. 8, I1 denotes a primary recoil impulse generated during the primary recoil, and I2 denotes a secondary recoil impulse generated during the secondary recoil.

The first recoil impulse I1 and the second recoil impulse I2 generated by the difference of the first preset time Δt are equal to each other.

Thus, if the first preset time Δt is reduced, the user feels that the first recoil impulse I1 and the second recoil impulse I2 occur almost simultaneously, so that the effect of offsetting the primary recoil impulse I1 and the secondary recoil impulse I2 can be seen. That is, as shown in FIG. 8B, an optimal time when recoil may be offset by reducing the first preset time Δt, is derived so that a recoil offsetting effect can be obtained.

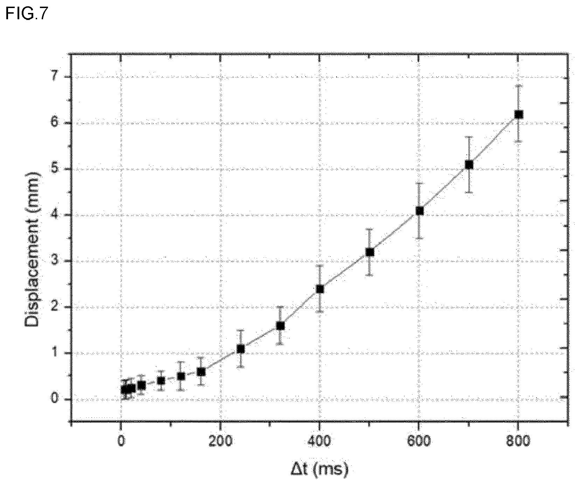

FIG. 7 is a graph showing a recoil displacement according to time when a current is applied to the solenoid coil, in the needleless syringe according to the first embodiment.

Referring to FIG. 7, an experiment was performed to measure the recoil distance while changing the first preset time in order to derive an optimal value of the first preset time Δt.

In the experiment, the first preset time Δt was gradually decreased from 800 ms, and the moving distance of the moving magnetic body was fixed to 60 mm. The weight of the moving magnetic body was decreased as the first preset time Δt decreased, so that impulse of the moving magnetic body 90 was kept constant.

Here, the recoil distance is the movement distance of the needleless syringe when the drug is injected once. The shorter the recoil distance, the less the recoil impulse felt by the user.

When the voltage applied to the solenoid coil 30 is increased, the movement speed of the moving magnetic body 90 is increased, so that the first preset time Δt can be reduced.

At this time, when the movement speed of the moving magnetic body 90 increases, impulse applied to the piston 40 increases, so that the mass of the moving magnetic body 90 relative to the movement speed needs to be reduced so as to keep the impulse constant. Because the mass of the moving magnetic body 90 can be adjusted in length or reduction in cross-sectional area, it is preferable to adjust the mass by forming a hole therein rather than adjusting the length.

In this experiment, the first preset time Δt was decreased by increasing the voltage applied to the solenoid coil 30, but the mass of the moving magnetic body 90 was reduced in order to maintain the impulse applied to the piston 40.

As a result of the above experiment, referring to FIG. 7, it can be seen that there is little change in the recoil distance when the first preset time Δt is 250 ms or less.

Thus, the first preset time Δt is 250 ms or less, and the optimal weight of the moving magnetic body 90 according to the decrease of the first preset time Δt is 100 g or less.

By setting the time for which the current is applied to the solenoid coil 30 to 250 ms or less, the recoil felt by the user when using the needleless syringe 100 is offset, so that convenience of use can be improved.

As described above, in the present invention, a current is periodically supplied to the solenoid coil 30 or the supply of current to the solenoid coil 30 is periodically cut off, so that the forward movement and the backward movement of the piston 40 can be repeated.

In addition, because a small amount of the drug can be repeatedly injected at high speed, a small amount of the drug can be injected into the entire skin several times in the field of skin care and the like.

In addition, in the present embodiment, since the piston 40 moves forward by the impact force, the piston 40 and the moving magnetic body 90 may move forward at a higher speed than when the piston 40 and the moving magnetic body 90 move forward integrally. Thus, electrical energy required to move the piston 40 forward can be further reduced.

In addition, since the recoil of the needleless syringe 100 may be minimized by adjusting the time when the current is applied to the solenoid coil 30, there is no need to add a separate recoil offset structure, so that the structure is simple and convenience of use can be improved.

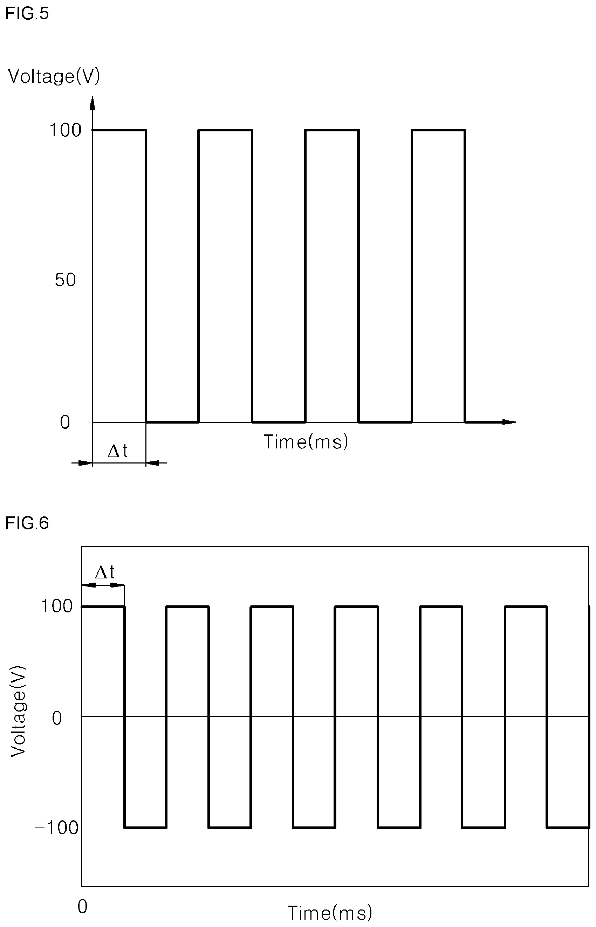

On the other hand, FIG. 5 is a graph showing an example of the current supply waveform applied to the solenoid coil of the needleless syringe according to the first embodiment.

Referring to FIG. 5, the current supply unit (not shown) applies a voltage of about 100V to the solenoid coil 30 for the first preset time Δt when the piston 40 moves forward, and the current supply unit (not shown) cuts off the voltage to the solenoid coil 30 when the piston 40 moves backward.

An example in which the time for which the voltage is cut off to the solenoid coil 30, is set to be the same as the time for which the voltage is applied to the solenoid coil 30, will be described.

The amount of voltage or time applied to the solenoid coil 30 may be set in various ways in consideration of the amount of the drug.

On the other hand, FIG. 6 is a graph showing another example of the current supply waveform applied to the solenoid coil of the needleless syringe according to the first embodiment.

Referring to FIG. 6, the current supply unit (not shown) repeatedly switches the direction of the current applied to the solenoid coil 30 at a preset period to periodically change the direction of the magnetic force generated in the solenoid coil 30.

An example in which the current supply unit (not shown) applies a voltage of about 100V to the solenoid coil 30 for the first preset time Δt when the moving magnetic body 90 moves forward and when the moving magnetic body 90 moves backward, the current supply unit (not shown) applies a voltage of about -100V for the first preset time Δt, will be described.

Thus, when a voltage of 100V is applied to the solenoid coil 30, a magnetic force is generated in a direction in which the moving magnetic body 90 moves forward, so that the moving magnetic body 90 can move forward.

In addition, when a voltage of -100V is applied to the solenoid coil 30, a magnetic force is generated in a direction in which the moving magnetic body 90 moves backward, so that the moving magnetic body 90 can move backward.

In addition, the present invention is not limited thereto, and the current supply unit (not shown) may include a capacitor (not shown) that stores the current supplied from an external power supply source, supplies the stored current to the solenoid coil 30 when the piston 40 moves forward and cuts off the supply of current to the solenoid coil 30 when the piston 40 moves backward, and a DC power supply unit that supplies the current supplied from the external power supply source when the piston 40 moves backward, to the solenoid coil 30.

That is, the current supply unit (not shown) repeatedly switches the direction of the current applied to the solenoid coil 30 at a preset cycle, but when the piston 40 moves forward, the current supply unit (not shown) supplies the current stored in the capacitor (not shown), and when the piston 40 moves backward, the current supply unit (not shown) supplies a current from the DC power supply unit to increase the movement speed during the forward movement of the piston 40.

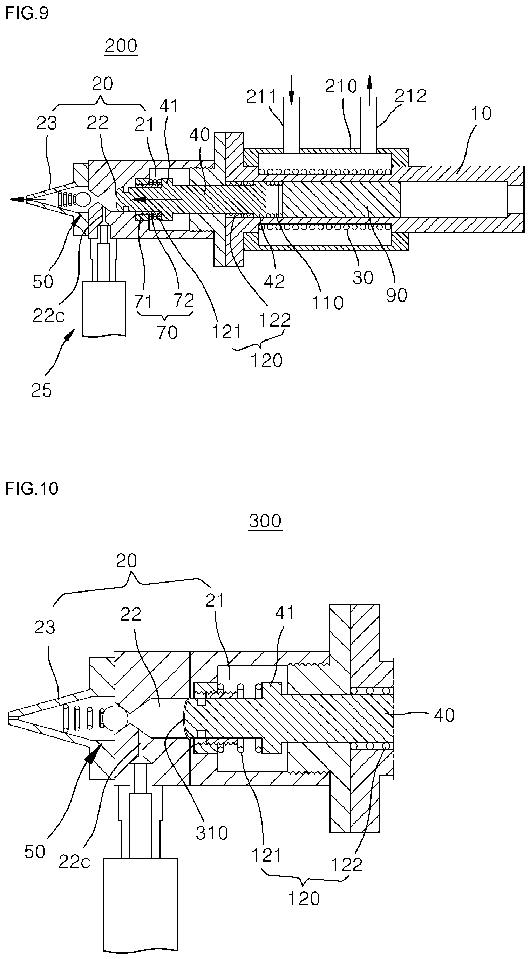

FIG. 9 is a longitudinal sectional view showing a configuration in which a cooling chamber is provided in a needleless syringe according to a second embodimentnot falling into the scope of claim 1.

Referring to FIG. 9, a needleless syringe 200 according to the second embodiment is different from that of the first embodiment in that the needleless syringe 200 according to the second embodiment further includes a cooling chamber 210 for cooling heat generated in the solenoid coil 30 through a cooling fluid, and the rest of the configuration and operation thereof is similar to those the first embodiment, and thus, a description of the similar configurations will be omitted, and different configurations will be mainly described below.

The cooling chamber 210 is detachably coupled to the outside of the body 10.

The cooling chamber 210 is installed to surround the solenoid coil 30 on the outer circumferential surface of the body 10. A cooling fluid supply pipe 211 and a cooling fluid discharge pipe 212 are coupled to the cooling chamber 210.

The cooling fluid supply pipe 611 is a flow path for supplying a cooling fluid from the outside to the cooling chamber 210. The cooling fluid discharge pipe 212 is a flow path for discharging the cooling fluid of the cooling chamber 210 to the outside. An opening/closing valve (not shown) may be provided in the cooling fluid supply pipe 211 and the cooling fluid discharge pipe 212, respectively.

In addition, in the present embodiment, a cooling fluid is used to cool the solenoid coil 30, and water or air is used as the cooling fluid. However, the present invention is not limited, and a conduction cooling method or the like may be used.

The needleless syringe 200 according to the second embodiment configured as described above is provided with the cooling chamber 210 for cooling the solenoid coil 30, so that heat of the solenoid coil 30 can be absorbed and the solenoid coil can be kept at a constant temperature and thus, the magnetic force can be prevented from being weakened by the heat generated by the solenoid coil 30.

FIG. 10 is a longitudinal cross-sectional view showing a configuration in which a piston cover is provided in a needleless syringe according to a third embodiment falling into the scope of claim 1.

Referring to FIG. 10, a needleless syringe 300 according to the third embodiment of the present invention is different from those of the first and second embodiments in that a piston cover 310 is provided between the cylinder 20 and the piston 40, and the reset of configurations and operations are similar to those of the above embodiments, and thus only different configurations will be described.

The piston cover 310 is applicable to both the first and second embodiments.

The piston cover 310 is fixedly installed on the cylinder 20. The piston cover 810 is provided inside the cylinder 20 and is disposed to cover the end of the piston 40. The piston cover 310 may be fitted and coupled to the inside of the cylinder 20, and may be fixed to the inner circumferential surface of the cylinder 20 by bonding or coupling.

The piston cover 310 is formed of a stretchable material so as to be stretched forward by the piston 40 when the piston 40 moves forward and to be restored when the piston 40 moves backward. In the present embodiment, the piston cover 310 will be described as an example of a rubber diaphragm.

The piston cover 310 may prevent the drug from being directly deposited on the end of the piston 40.

Thus, there is no need for the user to wipe the end of the piston 40.

In addition, because the piston cover 310 is provided in the cylinder 20, the piston cover 310 is also replaceable when the cylinder 20 is replaced.

The invention is defined by the appended claims.

### INDUSTRIAL APPLICABILITY

According to the present invention, there is an advantage that a needleless syringe can inject a predetermined drug repeatedly at high speed.

## Claims

1. A needleless syringe comprising:
a body (10) formed in a hollow shape;
a solenoid coil (30) wound around an outer circumferential surface of the body (10);
a cylinder (20) coupled to the body to be in communication with an open front surface of the body and including a drug accommodating portion for accommodating a drug, and a nozzle portion for discharging the drug accommodated in the drug accommodating portion to a front;
a moving magnetic body (90), which is inserted into the body in a longitudinal direction and moves forward by a magnetic force generated when a current is applied to the solenoid coil (30);
a piston (40), which is inserted in front of the moving magnetic body (90) inside the body and is provided to penetrate the body and the cylinder (20), and moves forward by an impact force applied by the moving magnetic body (90) when the moving magnetic body (90) moves forward, to pressurize the drug in the drug accommodating portion toward the nozzle portion;
a nozzle portion opening/closing valve (50), which is provided to open and close a passage hole between the nozzle portion and the drug accommodating portion, is pushed by a fluidic pressure applied by the drug from the drug accommodating portion when the piston (40) moves forward, to open the passage hole, and when the fluidic pressure is released, the nozzle portion opening/closing valve (50) is elastically restored to close the passage hole; and
a forward/backward driving unit repeating forward and backward movement of the piston (40) by repeating the supply and cut off of a current to the solenoid coil (30) at a preset period,
wherein the forward/backward driving unit comprises:
a current supply unit, which is configured to repeatedly supply a current to the solenoid coil (30) to move the moving magnetic body (90) forward,
**characterized in that** the needleless syringe further comprises a piston cover (310) provided inside the cylinder (20), configured to cover an end of the piston (40) and formed of a stretchable material so as to be stretched when the piston (40) moves forward and to be restored when the piston moves backward while applying an elastic force to the piston (40) backward.

2. The needleless syringe of claim 1, wherein the current supply unit is configured to cut off the current after applying the current to the solenoid coil (30) for a first preset time, and a mass of the moving magnetic body (90) is 100 g or less, and the first preset time is set to 250 ms or less.

3. The needleless syringe of claim 1, wherein the piston cover (310) is fixedly installed on the cylinder (20).

4. The needleless syringe of claim 1, wherein the piston cover (310) is replaceable when the cylinder (20) is replaced.

5. The needleless syringe of claim 1, wherein the piston (40) has a first flange portion (41) protruding in a radial direction from an outer circumferential surface of a front portion located inside the cylinder (20).

6. The needleless syringe of claim 1, further comprising a blocker (70) provided between the piston and the cylinder and limiting a forward movement distance of the piston when the first flange portion is blocked during forward movement of the piston.

7. The needleless syringe of claim 6, wherein the blocker (70) comprises a ring-shaped fixed blocker (71) fixedly installed on an inner circumferential surface of the cylinder (20) and having a female thread formed on an inner circumferential surface of the fixed blocker (71).

8. The needleless syringe of claim 1, wherein the drug accommodating portion is formed in a shape of a diverging nozzle including a reduced portion whose cross-sectional area decreases toward the front and an enlarged portion extending from the reduced portion to increase the cross-sectional area again, and the reduced portion has a drug supply hole in which the drug is supplied from an outside by a pressure difference generated during backward movement of the piston (40).

9. The needleless syringe of claim 1, wherein the nozzle portion opening/closing valve (50) comprises a ball installed in the passage hole and an elastic member installed in the nozzle portion to support the ball.

10. The needleless syringe of claim 1, further comprising a cooling chamber (210) provided to surround an outside of the solenoid coil (30) from an outside of the body (10), to absorb heat generated in the solenoid coil (30) through a cooling fluid and to cool the solenoid coil (30).

## Patentansprüche

1. Nadellose Spritze, die aufweist:
einen Körper (10), der eine hohle Form hat;
eine Magnetspule (30), die um eine äußere Umfangsfläche des Körpers (10) gewickelt ist;
einen Zylinder (20), der mit dem Körper gekoppelt ist, um mit einer offenen Vorderfläche des Körpers in Verbindung zu stehen, und der einen Arzneimittelaufnahmeabschnitt zur Aufnahme eines Arzneimittels und einen Düsenabschnitt zur Abgabe des in dem Arzneimittelaufnahmeabschnitt aufgenommenen Arzneimittels nach vorne umfasst;
einen sich bewegenden magnetischen Körper (90), der in einer Längsrichtung in den Körper eingesetzt ist und sich durch eine Magnetkraft vorwärts bewegt, die erzeugt wird, wenn ein Strom an die Magnetspule (30) angelegt wird;
einen Kolben (40), der vor dem sich bewegenden magnetischen Körper (90) im Inneren des Körpers eingesetzt ist und vorgesehen ist, um den Körper und den Zylinder (20) zu durchdringen, und der sich durch eine Stoßkraft vorwärts bewegt, die durch den sich bewegenden magnetischen Körper (90) ausgeübt wird, wenn sich der sich bewegende magnetische Körper (90) vorwärts bewegt, um das Arzneimittel in dem Arzneimittelaufnahmeabschnitt in Richtung des Düsenabschnitts unter Druck zu setzen; ein Düsenabschnitt-Öffnungs-/Schließventil (50), das vorgesehen ist, um ein Durchgangsloch zwischen dem Düsenabschnitt und dem
Arzneimittelaufnahmeabschnitt zu öffnen und zu schließen, wird durch einen Fluiddruck gedrückt, der durch das Arzneimittel von dem Arzneimittelaufnahmeabschnitt ausgeübt wird, wenn sich der Kolben (40) vorwärts bewegt, um das Durchgangsloch zu öffnen,
und wenn der Fluiddruck aufgehoben wird, wird das Düsenabschnitt-Öffnungs-/Schließventil (50) elastisch zurückgestellt, um das Durchgangsloch zu schließen; und eine Vorwärts-/Rückwärts-Antriebseinheit, die die Vorwärts- und Rückwärtsbewegung des Kolbens (40) durch wiederholtes Zuführen und Abschalten eines Stroms zur Magnetspule (30) in einer voreingestellten Periode wiederholt,
wobei die Vorwärts/Rückwärts-Antriebseinheit aufweist:
eine Stromzufuhreinheit, die so konfiguriert ist, dass sie der Magnetspule (30) wiederholt einen Strom zuführt, um den sich bewegenden magnetischen Körper (90) vorwärts zu bewegen,
**dadurch gekennzeichnet, dass** die nadellose Spritze ferner eine Kolbenabdeckung (310) aufweist, die im Inneren des Zylinders (20) vorgesehen ist, so konfiguriert ist, dass sie ein Ende des Kolbens (40) abdeckt, und aus einem dehnbaren Material besteht, so dass sie gedehnt wird, wenn sich der Kolben (40) vorwärts bewegt und zurückgestellt wird, wenn sich der Kolben rückwärts bewegt, während eine elastische Kraft nach hinten auf den Kolben (40) ausgeübt wird.

2. Nadellose Spritze nach Anspruch 1, wobei die Stromzufuhreinheit so konfiguriert ist, dass sie den Strom unterbricht, nachdem der Strom für eine erste voreingestellte Zeit an die Magnetspule (30) angelegt wurde, und eine Masse des sich bewegenden magnetischen Körpers (90) 100 g oder weniger beträgt und die erste voreingestellte Zeit auf 250 ms oder weniger eingestellt ist.

3. Nadellose Spritze nach Anspruch 1, wobei die Kolbenabdeckung (310) fest an dem Zylinder (20) installiert ist.

4. Nadellose Spritze nach Anspruch 1, wobei die Kolbenabdeckung (310) austauschbar ist, wenn der Zylinder (20) ersetzt wird.

5. Nadellose Spritze nach Anspruch 1, wobei der Kolben (40) einen ersten Flanschabschnitt (41) aufweist, der in einer radialen Richtung von einer äußeren Umfangsfläche eines im Inneren des Zylinders (20) angeordneten vorderen Abschnitts vorsteht.

6. Nadellose Spritze nach Anspruch 1, der ferner einen Blocker (70) aufweist, der zwischen dem Kolben und dem Zylinder vorgesehen ist und eine Vorwärtsbewegungsstrecke des Kolbens begrenzt, wenn der erste Flanschabschnitt während der Vorwärtsbewegung des Kolbens blockiert ist.

7. Nadellose Spritze nach Anspruch 6, wobei der Blocker (70) einen ringförmigen festen Blocker (71) aufweist, der fest an einer inneren Umfangsfläche des Zylinders (20) installiert ist und ein Innengewinde aufweist, das an einer inneren Umfangsfläche des festen Blockers (71) ausgebildet ist.

8. Nadellose Spritze nach Anspruch 1, wobei der Arzneimittelaufnahmeabschnitt in Form einer divergierenden Düse ausgebildet ist, die einen verringerten Abschnitt, dessen Querschnittsfläche nach vorne hin abnimmt, und einen vergrößerten Abschnitt umfasst, der sich von dem verringerten Abschnitt aus erstreckt, um die Querschnittsfläche wieder zu vergrößern, und der verringerte Abschnitt ein Arzneimittelzuführungsloch aufweist, in das das Arzneimittel von außen durch eine Druckdifferenz zugeführt wird, die während der Rückwärtsbewegung des Kolbens (40) erzeugt wird.

9. Nadellose Spritze nach Anspruch 8, wobei das Düsenabschnitt-Öffnungs-/Schließventil (50) eine Kugel, die in dem Durchgangsloch installiert ist, und ein elastisches Element aufweist, das in dem Düsenabschnitt installiert ist, um die Kugel zu stützen.

10. Nadellose Spritze nach Anspruch 1, die ferner eine Kühlkammer (210) aufweist, die so vorgesehen ist, dass sie eine Außenseite der Magnetspule (30) von einer Außenseite des Körpers (10) umgibt, um in der Magnetspule (30) erzeugte Wärme durch ein Kühlfluid zu absorbieren und die Magnetspule (30) zu kühlen.

## Revendications

1. Seringue sans aiguille, comprenant :
un corps (10) de forme creuse ;
une bobine de solénoïde (30) enroulée autour d'une surface circonférentielle extérieure du corps (10) ;
un cylindre (20) raccordé au corps de manière à communiquer avec une surface avant ouverte du corps, et comportant une partie de réception de médicament pour recevoir un médicament, et une partie de buse pour refouler vers l'avant le médicament reçu dans la partie de réception de médicament ;
un corps magnétique mobile (90), inséré dans le corps dans la direction longitudinale et se déplaçant vers l'avant sous l'effet d'une force magnétique générée lorsqu'un courant est appliqué à la bobine de solénoïde (30) ;
un piston (40), inséré devant le corps magnétique mobile (90) à l'intérieur du corps et prévu pour traverser le corps et le cylindre (20), et se déplaçant vers l'avant sous l'effet d'une force d'impact exercée par le corps magnétique mobile (90) lorsque le corps magnétique mobile (90) se déplace vers l'avant, pour mettre sous pression le médicament présent dans la partie de réception de médicament vers la partie de buse ;
une soupape d'ouverture/fermeture de partie de buse (50), qui est prévue pour ouvrir et fermer un orifice de passage entre la partie de buse et la partie de réception de médicament, est poussée par une pression fluidique exercée par le médicament à partir de la partie de réception de médicament lorsque le piston (40) se déplace vers l'avant, pour ouvrir l'orifice de passage, ladite soupape d'ouverture/fermeture de partie de buse (50) étant rappelée élastiquement pour obturer l'orifice de passage lorsque la pression fluidique est relâchée ; et
une unité d'entraînement vers l'avant/vers l'arrière répétant d'avance et de recul du piston (40) en alternant l'alimentation et la coupure du courant vers la bobine de solénoïde (30) à intervalle prédéfini,
où l'unité d'entraînement vers l'avant/vers l'arrière comprend :
une unité d'alimentation en courant, prévue pour fournir de manière répétée un courant à la bobine de solénoïde (30) afin de déplacer le corps magnétique mobile (90) vers l'avant, **caractérisée en ce que** ladite seringue sans aiguille comprend en outre un couvercle de piston (310) disposé à l'intérieur du cylindre (20), prévu pour recouvrir une extrémité du piston (40) et constitué d'un matériau extensible lui permettant d'être étiré lorsque le piston (40) se déplace vers l'avant, et de reprendre sa forme initiale lorsque le piston se déplace vers l'arrière en exerçant sur le piston (40) une force élastique vers l'arrière.

2. Seringue sans aiguille selon la revendication 1, où l'unité d'alimentation en courant est prévue pour couper le courant après avoir appliqué le courant à la bobine de solénoïde (30) pendant une première durée prédéfinie, et où la masse du corps magnétique mobile (90) est inférieure ou égale à 100 g, et la première durée prédéfinie est réglée sur 250 ms ou moins.

3. Seringue sans aiguille selon la revendication 1, où le couvercle de piston (310) est monté de manière fixe sur le cylindre (20).

4. Seringue sans aiguille selon la revendication 1, où le couvercle de piston (310) est remplaçable lors du remplacement du cylindre (20).

5. Seringue sans aiguille selon la revendication 1, où le piston (40) comporte une première partie de bride (41) faisant saillie dans une direction radiale à partir de la surface circonférentielle extérieure d'une partie avant située à l'intérieur du cylindre (20).

6. Seringue sans aiguille selon la revendication 1, comprenant en outre un élément de blocage (70) prévu entre le piston et le cylindre et limitant une course de déplacement vers l'avant du piston lorsque la première partie de bride est bloquée pendant le mouvement d'avance du piston.

7. Seringue sans aiguille selon la revendication 6, où l'élément de blocage (70) comprend un élément de blocage fixe (71) de forme annulaire monté de manière fixe sur une surface circonférentielle intérieure du cylindre (20) et présentant un filet femelle formé sur une surface circonférentielle intérieure dudit élément de blocage fixe (71).

8. Seringue sans aiguille selon la revendication 1, où la partie de réception de médicament se présente sous la forme d'une buse divergente comportant une partie réduite dont la surface de section transversale diminue vers l'avant, et une partie agrandie s'étendant à partir de la partie réduite pour accroître à nouveau la surface de section transversale, et où la partie réduite présente un orifice d'alimentation en médicament dans lequel le médicament est introduit depuis l'extérieur sous l'effet d'une différence de pression générée pendant le mouvement vers l'arrière du piston (40).

9. Seringue sans aiguille selon la revendication 1, où la soupape d'ouverture/fermeture de partie de buse (50) comprend une bille installée dans l'orifice de passage et un élément élastique installé dans la partie de buse pour supporter la bille.

10. Seringue sans aiguille selon la revendication 1, comprenant en outre une chambre de refroidissement (210) prévue pour entourer l'extérieur de la bobine de solénoïde (30) depuis l'extérieur du corps (10), afin d'absorber la chaleur générée dans la bobine de solénoïde (30) par un fluide de refroidissement et refroidir la bobine de solénoïde (30).
